# EUROPÄISCHE PATENTSCHRIFT

(11) **EP 0 471 217 B1**
(45) Veröffentlichungstag und Bekanntmachung des Hinweises auf die Patenterteilung: **03.09.1997**
(21) Anmeldenummer: 91112365.1
(22) Anmeldetag: 24.07.1991
(51) Int. Cl.: A61K 9/48, A23L 1/308

(54) **Mittel zur oralen Einnahme**
Composition for oral intake
Composition d'absorption orale

(30) Priorität: 16.08.1990 DE 4025912
(43) Veröffentlichungstag der Anmeldung: 19.02.1992
(73) Patentinhaber: Ratjen, Werner, D-24220 Flintbek (DE); Willmen, Hans Rainer Prof. Dr. med., D-41515 Grevenbroich (DE)
(72) Erfinder: Ratjen, Werner, D-24220 Flintbek (DE); Willmen, Hans Rainer Prof. Dr. med., D-41515 Grevenbroich (DE)
(74) Vertreter: Vollmann, Heiko, Dipl.-Ing.

(56) Entgegenhaltungen:
- EP-A- 0 043 317
- EP-A- 0 241 710
- EP-A- 0 317 079
- WO-A-90/01879
- US-A- 4 401 682
- PATENT ABSTRACTS OF JAPAN vol. 6, no. 44 (C-95)(922) 19. März 1982

## Beschreibung

Die Erfindung betrifft ein Mittel zur oralen Einnahme mit einem im Magen lösbaren und den Inhalt freigebenden Behältnis gemäß den im Oberbegriff des Anspruchs 1 angegebenen Merkmalen sowie ein Verfahren zur Herstellung eines solchen Mittels.

Es sind zahlreiche Versuche unternommen worden auf medikamentösem Wege überflüssige Fettanreicherungen im menschlichen Körper, wie sie heutzutage bei zahlreichen Menschen als eine Art Zivilisationskrankheit zu beobachten sind, abzubauen bzw. deren Entstehung zu verhindern. Es gibt sogenannte Appetitzügler, die den Körper auf biochemischem Wege eine Abneigung zur Nahrungsaufnahme (Appetitlosigkeit) zu suggerieren versuchen. Diese Mittel haben zum Teil erhebliche schädliche Nebenwirkungen.

Neben den zahlreichen bekannten Diätvorschlägen gibt es auch mechanische und elektromechanische Mittel, mit denen ein gezielter Fettabbau bzw. Muskelaufbau erfolgen soll. Die Wirkungsweise solcher Mittel ist sehr zweifelhaft.

Die einfache Reduzierung der Nahrungsaufnahme erfordert ein Maß an Selbstbeherrschung, welches viele Menschen auf Dauer nicht in der Lage sind aufzubringen.

Aus EP-A-0 317 079 ist ein Mittel bekannt, bei dem die Inhaltsstoffe praktisch ohne Verbund, jedoch in gepreßter Form in Kapseln eingebracht oder zu Tabletten gepreßt werden. Nach dem Einnehmen löst sich die Kapsel bzw. die gepreßte Tablette im Magen auf, die Inhaltsstoffe bilden eine Quellmasse, die im Magen befindliche Flüßigkeit bindet und über den Verdauungstrakt abgebaut bzw. wieder ausgeschieden wird. Da das Mittel keinerlei Brennwert hat, dem Körper jedoch ein Sättigungsgefühl aufgrund der im Magen befindlichen Quellmasse vorgetäuscht wird, kann die Nahrungsaufnahme ohne große Überwindung zum Zwecke der Gewichtsreduktion eingestellt bzw. vermindert werden. Da die Quellmasse im wesentlichen ungebunden ist, wird sie relativ schnell aus dem Magen in den weiteren Verdauungstrakt geleitet, weshalb das dadurch hervorgerufene Sättigungsgefühl nur vergleichsweise kurze Zeit anhält.

Aus EP-A-0 043 317 ist ein hydrophiler Stoff auf Polyurethan-Polyol-Basis bekannt, der die Eigenschaft hat, Wasser zu binden und auch zum therapeutischen und kosmetischen Einsatz vorgesehen ist. Als Mittel zur Gewichtsreduktion ist dieser Stoff jedoch auch aus den vorerwähnten Gründen nicht geeignet.

Weiterhin sind aus US-A-4,401,682 sowie WO,90/01879 Mittel basierend auf Zellulosefasern bekannt, denen weitere Ingredienzen zugegeben sind. Diese Zellulosefasern nebst Zusatzstoffen werden zu einem feinen Puder zerkleinert und mit Gelatine vermengt, so daß sich bei Einnahme dieser Mittel ebenfalls eine gallertartige Masse mit undefinierter Raumstruktur im Magen bildet, die vergleichsweise schnell abbaubar ist. Dies führt dazu, daß vergleichsweise große Mengen des Mittels in zeitlich kurzen Abständen eingenommen werden müssen, um das gewünschte Sättigungsgefühl zu erzeugen. Dies ist sowohl aus Kostengründen als auch wegen der mit erhöhtem Massendurchsatz auch steigenden Gefahr von Nebenwirkungen ungünstig.

Der Erfindung liegt daher die Aufgabe zugrunde, ein Mittel zur Gewichtsreduzierung zu schaffen, das unter Vermeidung der vorgenannten Nachteile, insbesondere der bei Appetitzüglern regelmäßig auftretenden Nebenwirkungen, bei einfacher Anwendung und billiger Herstellung auch ohne ärztlichen Beistand unproblematisch anwendbar ist.

Des weiteren soll ein Verfahren zur Herstellung eines solchen Mittels geschaffen werden.

Die erfindungsgemäße Lösung besteht in einem Mittel zur oralen Einnahme, das aus einem im Magen lösbaren und den Inhalt freigebenden Behältnis besteht, das mit einem nichttoxischen, bei seinem Freisetzen volumenvergrößernden und brennstoffarmen Stoff gefüllt ist, der innerhalb des Verdauungstraktes abbaubar oder durch den Verdauungstrakt abführbar ist. Im Unterschied zum Stand der Technik ist bei dem erfindungsgemäßen Mittel der Stoff durch einen Körper mit schwammartiger Struktur gebildet, der in komprimierter Form im Behältnis angeordnet und durch das Behältnis in dieser komprimierten Form gehalten wird.

Der verfahrensmäßige Teil der vorgenannten Aufgabe wird durch die in Anspruch 5 aufgeführten Merkmale gelöst. Die in den Unteransprüchen aufgeführten Merkmale stellen vorteilhafte Weiterbildungen der Erfindung dar.

Der besondere Vorteil des erfindungsgemäßen Mittels liegt darin, daß der im Magen freigesetzte Stoff einen Körper mit schwammartiger Struktur bildet, so daß er einerseits Flüßigkeit bindet und andererseits ein definiertes Volumen im Magen einnimmt. Aufgrund der körperhaften Form verbleibt der Stoff wesentlich länger im Magen als bei bekannten Mitteln dieser Art üblich. Diese vergleichsweise lange Verweildauer im Magen ist besonders vorteilhaft, da hierdurch für entsprechend lange Zeit ein Sättigungsgefühl erzeugt wird, ohne ständig Mittel ergänzen zu müssen.

Die Erfindung schafft ein Mittel, bei dessen Einnahme der Magen volumenmäßig gefüllt wird, wodurch dem Körper ein Sättigungsgefühl suggeriert wird. Der in dem Mittel befindliche Stoff ist so gewählt, daß, bezogen auf sein freies Volumen, er extrem brennstoffarm und gut körperverträglich ist. Der Stoff sollte ferner so beschaffen sein, daß beim Freisetzen im Magen eine erhebliche, vorzugsweise vielfache Volumenvergrößerung erfolgt, zudem muß der Stoff innerhalb des menschlichen Verdauungstraktes abbaubar oder über diesen abführbar sein. Derartige Stoffe sind bekannt und in den Unteransprüchen angegeben. Es können hier beispielsweise Polyurethanschaumstoff oder Zellulose (als Naturstoff) oder auch andere geeignete Stoffe eingesetzt werden.

Besonders bewährt hat sich Zelluloseschwamm mit einer Dichte von 0,01 kg/dm³ bis 0,05 kg/dm³, vorzugsweise 0,03 kg/dm³. Bevorzugt wird hier ein aus Alveolarzellulose bestehender Schwamm Verwendung finden, die durch Naturfasern, beispeilsweise Baumwolle verstärkt ist. Alveolarzellulose ist aus Holzpulpe hergestellte Zellulose. Dieser Zelluloseschwamm kann zum Einfüllen in das Behältnis, insbesondere eine Gelantinekapsel, eng gewickelt oder gepreßt werden. Dieser Stoff ist - soweit bisher bekannt - völlig ungefährlich für den menschlichen Körper und wird verhältnismäßig langsam abgebaut, so daß die Wirkung eines damit gefüllten Mittels relativ lange anhält.

Statt Zellulose können, wie oben erwähnt, auch andere geeignete Stoffe eingesetzt werden, wie z. B. Polyurethanschaumstoff. Hierbei wird vorteilhaft ein Schaumstoff mit einer Dichte von weniger 0,025 kg/dm³ eingesetzt.

Um zu erreichen, daß die Volumenvergrößerung erst im Magen erfolgt, ist es erforderlich, den Füllstoff in ein Behältnis einzusetzen, das sich im Magen auflöst, zumindest aber dort zerstört wird. Zweckmäßigerweise werden hierzu handelsübliche Gelantinekapseln eingesetzt, die einerseits eine genügende mechanische Stabilität aufweisen, um den Füllstoff in seiner komprimierten Form zu halten und andererseits billig in der Herstellung und seit Jahren mit Erfolg eingesetzt sind.

Die vorbeschriebenen Mittel sind bekanntermaßen gut verträglich und sehr kostengünstig in der Herstellung. Auch eine Einnahme dieser Mittel durch Kinder - sei es gewollt oder auch mißbräuchlich - ist weitgehend unproblematisch.

Technisch stellt es ein gewisses Problem dar, den Füllstoff zu komprimieren und in dieser komprimierten Form in das Behältnis einzuführen bzw. den Füllstoff direkt in dem Behältnis zu komprimieren. Die Erfindung sieht hierzu ein Verfahren vor, wie es in Anspruch 8 beschrieben ist. Dieses Verfahren eignet sich insbesondere beim Einsatz von Polyurethanschaumstoff oder Zelluloseschwamm als Wirkstoff, kann jedoch auch in Verbindung mit anderen Stoffen angewandt werden. Der Füllstoff wird dabei vor dem Einführen in das Behältnis eng aufgewickelt oder gepreßt, wodurch dieser komprimiert und auf ein Bruchteil seines freien Volumens reduziert wird. Der komprimierte Füllstoff wird dann anschließend in den einen Teil einer zweiteiligen Kapsel eingeführt, die abschließend durch den anderen übergreifenden Kapselteil in an sich bekannter Weise verschlossen wird. Dieses Verfahren hat sich in Vorversuchen besonders bewährt, da hierdurch eine sehr hohe Komprimierung des Füllstoffes möglich ist und dieses Verfahren auch großtechnisch anwendbar ist.

## Patentansprüche

1. Mittel zur oralen Einnahme mit einem im Magen lösbaren und den Inhalt freigebenden Behältnis, das mit einem beim Freisetzen volumenvergrößernden, nicht toxischen und brennstoffarmen Stoff gefüllt ist, der innerhalb des Verdauungstraktes abbaubar oder über diesen abführbar ist, dadurch gekennzeichnet, daß der Stoff durch einen Körper mit schwammartiger Struktur gebildet ist, der im komprimierter Form im Behältnis angeordnet und durch das Behältnis in dieser komprimierten Form gehalten wird.

2. Mittel nach Anspruch 1, dadurch gekennzeichnet, daß der Stoff aus Zellulose besteht.

3. Mittel nach Anspruch 1 oder 2, dadurch gekennzeichnet, daß das Behältnis durch eine vorzugsweise zweiteilige Gelantinekapsel gebildet wird.

4. Mittel nach einem der vorhergehenden Ansprüche, dadurch gekennzeichnet, daß das Behältnis mit einem komprimierten Zelluloseschwamm gefüllt ist.

5. Verfahren zur Herstellung eines Mittels nach einem der Ansprüche 1 bis 4, dadurch gekennzeichnet, daß ein Streifen des Stoffes unter Verdrängung eines Großteiles des darin befindlichen Gases eng aufgewickelt oder gepreßt wird und dann in dieser Form in den einen Teil einer zweiteiligen Kapsel eingeführt wird, wonach die Kapsel durch den zweiten Kapselteil verschlossen wird.

## Claims

1. A composition for oral intake with a receptacle which in the stomach can be dissolved and releases the contents, said receptacle being is filled with material which on releasing increases volume, is non-toxic, is of low calorific value and which can be degraded within and removed by the digestive tract, characterised in that the material is formed by a body with a sponge like structure which in the compressed form is arranged in the receptacle and is kept by the receptacle in this compressed form.

2. A composition according to claim 1, characterised in that the material is composed of cellulose.

3. A composition according to claim 1 or 2, characterized in that the receptacle is preferably formed by a two part gelatine capsule.

4. A composition according to one of the preceding claims, characterised in that the receptacle is filled with a compressed cellulose sponge.

5. A method for producing a composition according to one of claims 1 to 4, characterised in that a strip of the material with a displacement of a large part of the gas located therein is tightly wound up or pressed and then is introduced in this form into one part of the two part capsule, after which the capsule is closed by the second capsule part.

## Revendications

1. Composition pour prise par voie orale, comportant une enveloppe soluble dans l'estomac et pouvant y libérer son contenu, enveloppe qui est emplie d'une matière augmentant de volume lors de sa libération, qui n'est pas toxique et est peu calorique, qui peut être dégradée à l'intérieur du tube digestif ou peut être évacuée et excrétée par lui, composition caractérisée en ce que la matière est formée d'un corps à structure spongieuse, qui est disposé sous forme comprimée dans l'enveloppe et est maintenue sous cette forme comprimée par l'enveloppe.

2. Composition selon la revendication 1, caractérisée en ce que la matière est constituée par de la cellulose.

3. Composition selon la revendication 1 ou 2, caractérisée en ce que l'enveloppe est formée par une capsule en gélatine, qui est avantageusement en deux parties.

4. Composition selon l'une des revendications précédentes, caractérisée en ce que l'enveloppe est emplie d'une mousse ou éponge de cellulose comprimée.

5. Procédé de préparation d'une composition selon l'une des revendications 1 à 4, caractérisé en ce qu'une bande de la matière est enroulée de manière étroite ou est comprimée, avec expulsion d'une grande partie du gaz qui s'y trouve, puis est introduite sous cette forme, dans une partie d'une capsule en deux parties, après quoi la capsule est fermée par sa seconde partie.
